Europäisches Patentamt

European Patent Office

Office européen des brevets

(19)

(11) Veröffentlichungsnummer : **0 409 052 B1**

(12) **EUROPÄISCHE PATENTSCHRIFT**

(45) Veröffentlichungstag der Patentschrift :
**12.05.93 Patentblatt 93/19**

(51) Int. Cl.$^5$ : **A61K 37/02, A61K 37/64, C07K 15/00**

(21) Anmeldenummer : **90113186.2**

(22) Anmeldetag : **11.07.90**

(54) **Antikoagulanz enthaltendes Mittel.**

(30) Priorität : **15.07.89 DE 3923500**

(43) Veröffentlichungstag der Anmeldung :
**23.01.91 Patentblatt 91/04**

(45) Bekanntmachung des Hinweises auf die
Patenterteilung :
**12.05.93 Patentblatt 93/19**

(84) Benannte Vertragsstaaten :
**AT BE CH DE DK ES FR GB GR IT LI LU NL SE**

(56) Entgegenhaltungen :
**EP-A- 0 181 465**
**EP-A- 0 409 053**
**The Journal of Biological Chemistry, vol. 264, no. 24, 25 August 1989, p. 14463-14470 KAET-ZEL M.A. et al: "Differential tissue expression of three 35-kDa annexin calcium-dependent phospholipid-binding proteins."**

(73) Patentinhaber : **BOEHRINGER INGELHEIM INTERNATIONAL G.M.B.H.**
**W-6507 Ingelheim am Rhein (DE)**

(72) Erfinder : **Reutelingsperger, Christiaan, Dr.**
**Looiersgracht 17**
**NL-6211 JK Maastricht (NL)**

EP 0 409 052 B1

## Beschreibung

Gegenstand der vorliegenden Erfindung ist ein Mittel, das neben einem Antikoagulanz oder seinen Analoga bivalente Kationen enthält.

Der Blutgerinnungsmechanismus stellt sich als eine Kaskade enzymatischer Reaktionen dar, an deren Ende die Bildung von Thrombin steht, das letztendlich Fibrinogen in Fibrin umwandelt. Verschiedene prokoagulante Reaktionen wie beispielsweise die Aktivierung von Prothrombin durch die Faktoren Xa und Va werden katalysiert durch Phospholipidoberflächen, an die die Koagulationsfaktoren binden. Nicht jede Art der Phospholipide vermag die Gerinnung zu stimulieren. Die Ladung der Phosholipidoberfläche scheint das Ausmaß des Einflusses zu bestimmen. Negativ geladene Phospholipide wie Phosphatidylserin haben eine hohe prokoagulatorische Wirkung.

Bei den Proteinen, die an Phospholipide binden und mit Phospholipidoberflächen abhängigen Prozessen interferieren, gibt es eine Familie, die in ihrer Bindung an Phospholipide $Ca^{2+}$ abhängig sind.

Zu dieser Familie, die auch Annexine genannt wird, gehört neben Lipocortin I, Calpactin I, Protein II, Lipocortin III, p67-Calelectrin auch das Vascular Antikoagulant Protein (VAC$\alpha$ und VAC$\beta$) und IBC, PAP, PAPI, PP4, Endonexin II und Lipocortin V.

Die gemeinsamen strukturellen Merkmale der Annexine sind wahrscheinlich die Grundlagen für ihre ähnlichen $Ca^{2+}$ und Phospholipidbindungseigenschaften. Obwohl diese generelle Eigenschaft für alle Annexine gilt, besteht eine klare Individualität hinsichtlich ihren Affinität zu $Ca^{2+}$ und zu den verschiedenen Phospholipidarten.

Die physiologischen Funktionen der Annexine betreffen membranassoziierte Prozesse. Der grundlegende Mechanismus der gerinnungshemmenden Wirkung des VAC wurde als eine Hemmung der katalytischen Kapazität der Phospholipide durch die Bindung des VAC an ihre Oberfläche erkannt, wodurch die Bildung des gerinnungsfördernden Komplexes an ihrer Oberfläche verhindert wird.

Auch andere Annexine können die Blutgerinnung hemmen, doch scheint VAC der effektivste Inhibitor zu sein.

Die Aufgabe der vorliegenden Erfindung bestand darin, eine VAC-Präparation bereitzustellen, die wirksamer als reines VAC ist.

Bindungsstudien haben gezeigt, daß sich VAC Calcium-abhängig mit prokoagulatorischen Phospholipiden reversibel assoziiert.

Auch andere bivalente Kationen aus der Reihe $Cd^{2+}$, $Zn^{2+}$, $Mn^{2+}$ und $Co^{2+}$ beeinflussen die Assoziation positiv, jedoch nicht in dem Maße wie $Ca^{2+}$.

Gegenstand der vorliegenden Erfindung ist daher ein Mittel, das ein vaskulares Antikoagulanz aus der Gruppe der Annexine und seinen natürlich vorkommenden oder synthetisch oder gentechnisch hergestellten Derivaten oder Analoga und $Ca^{2+}$, und $Zn^{2+}$, enthält und gegebenenfalls Hilfs- und/oder Trägerstoffe und/oder Stabilisatoren.

Darüberhinaus wurde überraschenderweise gefunden, daß die VAC Adsorption an Phospholipide in Gegenwart von $Ca^{2+}$- und $Zn^{2+}$-Ionen außerordentlich positiv beeinflußt wird und daß hierdurch die blutgerinnungshemmende Wirkung von VAC verbessert wird.

Gegenstand der vorliegenden Erfindung ist daher auch ein Mittel, das eine Kombination aus einem vaskularen Antikoagulanz aus der Gruppe der Annexine und seinen natürlich vorkommenden oder synthetisch oder gentechnisch hergestellten Derivaten oder Analoga und $Ca^{2+}$ und $Zn^{2+}$ enthält und gegebenenfalls Hilfs- und/oder Trägerstoffe und/oder Stabilisatoren.

Die Konzentration des bivalenten Kations $Ca^{2+}$, in den erfindungsgemäßen Kombinationen beträgt zwischen 0,01 und 100 mM, vorzugsweise zwischen 0,03 und 10 mMol.

Zur Erzielung des synergistischen Effekts von $Zn^{2+}$-Ionen beträgt die Konzentration an Zink zwischen 0,1 und 100 µM, vorzugsweise zwischen 18 und 30 µM, besonders bevorzugt liegt sie im Bereich der normalen Plasma Zink Konzentration.

In Fällen, bei denen Calzium- und/oder Zink-Defizienz vorliegt, wird die Calzium- und/oder Zinkkonzentration mindestens so eingestellt, daß sie auf jeden Fall mindestens den normalen Plasmaspiegel erreicht.

Die erfindungsgemäß einsetzbaren Antikoagulantien, insbesondere VAC können nicht nur in freier Form, sondern auch in Form ihrer Salze, insbesondere ihrer pharmazeutisch annehmbaren Salze, vorliegen. Da sie mehrere Aminosäurereste mit freien Aminogruppen enthalten, können die erfindungsgemäßen Verbindungen z.B. in Form von Säureadditionssalzen vorliegen. Als Säureadditionssalze kommen insbesondere physiologisch verträgliche Salze mit üblichen, therapeutisch anwendbaren Säuren in Betracht; als anorganische Säuren sind die Halogenwasserstoffsäuren, wie die Chlorwasserstoffsäure, aber auch Schwefelsäure und Phosphor- bzw. Pyrophosphorsäure zu nennen; als organische Säuren sind in erster Linie Sulfonsäuren, wie die Benzol- oder p-Toluosulfonsäure oder Niederalkansulfonsäuren, wie Methansulfonsäure, sowie Carbonsäu-

2

ren, wie Essigsäuren, Milchsäure, Palmitin- und Stearinsäure, Äpfelsäure, Weinsäure, Ascorbinsäure und Citronensäure geeignet. Da die Verbindungen auch Aminosäurereste mit freien Carboxylgruppen enthalten, können sie auch als Metallsalz, insbesondere als Alkalimetall- oder Erdalkalimetallsalz, z.B. Natrium-, Calcium- oder Magnesiumsalz, oder auch als Ammoniumsalz, abgeleitet von Ammoniak oder einer physiologisch verträglichen, organischen stickstoffhaltigen Base, vorliegen. Da sie aber zugleich freie Carboxylgruppen und freie Aminogruppen enthalten, können sie auch als innere Salz vorliegen.

Die erfindungsgemäßen neuen Mittel können in Analogie zu VAC zur Therapie und Prophylaxe von Thrombosen und Embolien, einschließlich zur Prophylaxe von postoperativen Thrombosen, zur akuten Schock-Therapie (z. B. bei septischem oder polytraumatischem Schock), zur Therapie von Verbrauchskoagulopathien insbesondere in den Fällen, bei denen Calzium- und/oder Zink-Defizienz vorliegt, bei Hämodialysen, Hämoseparationen, Blutkonserven und im extrakorporalen Kreislauf verwendet werden.

Auch bei allen intravasalen Manipulationen, bei denen Beschädigungen der Gefäßwände auftreten können und dadurch in manchen Fällen die Bildung von Thromben ausgelöst werden kann, können die erfindungsgemäßen Mittel therapeutisch und/oder prophylaktisch eingesetzt werden. Zu diesen intravasalen Manipulationen gehören beispielsweise die Ballonkatheterisierung, arteriöse/venöse Prothesen und die Entfernung arteriosklerotische Plaques beispielsweise durch Laserstrahlen.

Die Mittel können z. B. parenteral, wie intravenös, intracutan, subcutan oder intramuskulär, oder topisch verabreicht werden.

Die Dosierung hängt vom Zweck der Therapie bzw. Prophylaxe ab. Die Größe der Einzeldosen sowie das Verabreichungsschema kann am besten anhand einer individuellen Beurteilung des jeweiligen Krankheitsfalles bestimmt werden: die dazu erforderlichen Methoden zur Bestimmung von relevanten Faktoren sind dem Fachmann geläufig. Im Normalfall liegt bei einer Injektion die therapeutisch wirksame Menge der erfindungsgemäßen Mittel im Dosisbereich von 0,005 bis 0,1 mg/kg Körpergewicht. Bevorzugt wird der Bereich von 0,01 bis 0,05 mg/kg Körpergewicht. Die Verabreichung erfolgt durch intravenöse, intramuskuläre oder subcutane Injektion. Dementsprechend enthalten pharmazeutische Präparate zur parenteralen Verabreichung in Einzeldosis-Form in Abhängigkeit von der Applikationsart pro Dosis 0,4 bis 7,5 mg des Antikoagulanz. Neben der erfindungsgemäßen Kombination enthalten diese pharmazeutischen Zusammensetzungen gegebenenfalls noch einen Puffer, z. B. einen Phosphatpuffer, der den pH-Wert zwischen 3,5 und 8 halten soll, und ferner Natriumchlorid, Mannit oder Sorbit zur Einstellung der Isotonie. Sie können in gefriergetrockneter oder gelöster Form vorliegen, wobei Lösungen ein antibakteriell wirkendes Konservierungsmittel, z. B. 0,2 bis 0,3 % 4-Hydroxybenzoesäuremethylester oder -ethylester, enthalten können. Ein Präparat für die topische Anwendung kann als wässrige Lösung, Lotion oder Gelee, ölige Lösung oder Suspension, oder fetthaltige oder insbesondere Emulsions-Salbe vorliegen. Ein Präparat in Form einer wässrigen Lösung erhält man beispielsweise dadurch, daß man die erfindungsgemäße Kombination in einer wässrigen Pufferlösung von pH 4 bis 6,5 löst und gewünschtenfalls einen weiteren Wirkstoff, z. B. ein Antiinflammatorikum, und/oder ein polymeres Haftmittel, z. B. Polyvinylpyrrolidon, und/oder ein Konservierungsmittel zufügt. Die Konzentration des Wirkstoffs beträgt 0,1 bis 1,5 mg, vorzugsweise 0,25 bis 1,0 mg, in 10 ml einer Lösung bzw. 10 g eines Geles.

Eine ölige Applikationsform für die topische Verabreichung erhält man beispielsweise durch Suspendieren der erfindungsgemäßen Kombination in einem Oel, gegebenfalls unter Zusatz von Quellmitteln, wie Aluminiumstearat, und/oder grenzflächenaktiven Mitteln (Tensiden), deren HLB-Wert ("hydrophilic-lipophilic-balance") unter 10 liegt, wie Fettsäuremonoester mehrwertiger Alkohole, z. B. Glycerinmonostearat, Sorbitanmonolaurat, Sorbitanmonostearat oder Sorbitanmonooleat. Eine fetthaltige Salbe erhält man z. B. durch Suspendieren der erfindungsgemäßen Kombination in einer streichbaren Fettgrundlage, gegebenenfalls unter Zusatz eines Tensids vom HLB-Wert unter 10. Eine Emulsionssalbe erhält man durch Verreiben einer wässerigen Lösung der erfindungsgemäßen in einer weichen, streichbaren Fettunterlage unter Zusatz eines Tensids, dessen HLB-Wert unter 10 liegt. Alle diese topischen Applikationsformen können auch Konservierungsmittel enthalten. Die Konzentration des Wirkstoffes beträgt 0,1 bis 1,5 mg, vorzugsweise 0,25 bis 1,0 mg, in etwa 10 g der Grundmasse.

Neben den oben beschriebenen und ihren analogen pharmazeutischen Zusammensetzungen, welche für einen direkten medizinischen Einsatz am Körper des Menschen oder eines Säugetieres bestimmt sind, betrifft die vorliegende Erfindung auch pharmazeutische Zusammensetzungen und Präparate (wenn nicht anders angegeben in der vorliegenden Anmeldung als Mittel bezeichnet) zur medizinischen Anwendung außerhalb des lebenden Körpers des Menschen oder der Säugetiere. Solche Zusammensetzungen und Präparate verwendet man in erster Linie als gerinnungshemmenden Zusatz zu Blut, welches außerhalb des Körpers einer Zirkulation oder Behandlung (z.B. Extrakorporaler Kreislauf oder Dialyse in künstlichen Nieren), Konservierung oder Modifizierung (z.B. Hämoseparation) unterzogen wird. In ihrer Zusammensetzung sind derartige Präparate, wie Vorratslösungen oder auch Zubereitungen in Einzeldosis-Form, den oben beschriebenen Injektionspräparaten ähnlich; zweckmäßigerweise wird aber die Wirkstoffmenge bzw. -konzentration auf das Volumen des zu behandelnden Blutes bezogen. Je nach dem spezifischen Zweck beträgt die geeignete Dosis 0,01 bis 1,0 mg Wirkstoff/l Blut, wobei die obere Grenze sowohl des Wirkstoffs als auch der bivalenten Kationen ohne Gefahr noch überschritten werden darf.

Die vorliegende Erfindung betrifft im Einzelnen: Mittel, die ein vaskulares Antikoagulanz aus der Gruppe der Annexine und seinen natürlich vorkommenden oder synthetisch oder gentechnisch hergestellten Derivaten oder Analoga, $Ca^{2+}$, $Zn^{2+}$ und gegebenenfalls Hilfs- und/oder Trägerstoffe und/oder Stabilisatoren enthalten, bei denen das vaskulare Antikoagulanz vorzugsweise der Formel

```
    1                        5                           10                              15
    Met Ala Gln Val Leu Arg Gly Thr Val Thr Asp Phe Pro Gly Phe


                            20                          25                              30
    Asp Glu Arg Ala Asp Ala XX  Thr Leu Arg Lys Ala Met Lys Gly


                            35                          40                              45
    Leu Gly Thr Asp Glu Glu Ser Ile Leu Thr Leu Leu Thr Ser Arg


                            50                          55                              60
    Ser Asn Ala Gln Arg Gln Glu Ile Ser Ala Ala Phe Lys Thr Leu


                            65                          70                              75
    Phe Gly Arg Asp Leu Leu Asp Asp Leu Lys Ser Glu Leu Thr Gly


                            80                          85                              90
    Lys Phe Glu Lys Leu Ile Val Ala Leu Met Lys Pro Ser Arg Leu


                            95                          100                             105
    Tyr Asp Ala Tyr Glu Leu Lys His Ala Leu Lys Gly Ala Gly Thr


                            110                         115                             120
    Asn Glu Lys Val Leu Thr Glu Ile Ile Ala Ser Arg Thr Pro Glu


                            125                         130                             135
    Glu Leu Arg Ala Ile Lys Gln Val Tyr Glu Glu Glu Tyr Gly Ser


                            140                         145                             150
    Ser Leu Glu Asp Asp Val Val Gly Asp Thr Ser Gly Tyr Tyr Gln
```

4

155　　　　　　　　　　　160　　　　　　　　　　　165
Arg Met Leu Val Val Leu Leu Gln Ala Asn Arg Asp Pro Asp Ala

170　　　　　　　　　　　175　　　　　　　　　　　180
Gly Ile Asp Glu Ala Gln Val Glu Gln Asp Ala Gln Ala Leu Phe

185　　　　　　　　　　　190　　　　　　　　　　　195
Gln Ala Gly Glu Leu Lys Trp Gly Thr Asp Glu Glu Lys Phe Ile

200　　　　　　　　　　　205　　　　　　　　　　　210
Thr Ile Phe Gly Thr Arg Ser Val Ser His Leu Arg Lys Val Phe

215　　　　　　　　　　　220　　　　　　　　　　　225
Asp Lys Tyr Met Thr Ile Ser Gly Phe Gln Ile Glu Glu Thr Ile

230　　　　　　　　　　　235　　　　　　　　　　　240
Asp Arg Glu Thr Ser Gly Asn Leu Glu Gln Leu Leu Leu Ala Val

245　　　　　　　　　　　250　　　　　　　　　　　255
Val Lys Ser Ile Arg Ser Ile Pro Ala Tyr Leu Ala Glu Thr Leu

260　　　　　　　　　　　265　　　　　　　　　　　270
Tyr Tyr Ala Met Lys Gly Ala Gly Thr Asp Asp His Thr Leu Ile

275　　　　　　　　　　　280　　　　　　　　　　　285
Arg Val Met Val Ser Arg Ser Glu Ile Asp Leu Phe Asn Ile Arg

290　　　　　　　　　　　295　　　　　　　　　　　300
Lys Glu Phe Arg Lys Asn Phe Ala Thr Ser Leu Tyr Ser Met Ile

305　　　　　　　　　　　310　　　　　　　　　　　315
Lys Gly Asp Thr Ser Gly Asp Tyr Lys Lys Ala Leu Leu Leu Leu

320
Cys Gly Glu Asp Asp　　*

entspricht, wobei XX für Glu oder Asp steht und gegebenenfalls an Position 1 das Methionin abgespalten ist und das Alanin an Position 2 gegebenenfalls blockiert ist und/oder wobei gegebenenfalls Aggregationen durch beispielsweise intermolekulare Disulfidbrücken zwischen den Cysteinen an Position 316 vorliegen oder seinen biologisch aktiven Varianten oder Derivaten entspricht oder der Formel

```
       1                    5                    10                   15
      Met Ala Trp Trp Lys Ala Trp Ile Glu Gln Glu Gly Val Thr Val

                          20                   25                   30
      Lys Ser Ser Ser His Phe Asn Pro Asp Pro Asp Ala Glu Thr Leu

                          35                   40                   45
      Tyr Lys Ala Met Lys Gly Ile Gly Thr Asn Glu Gln Ala Ile Ile

                          50                   55                   60
      Asp Val Leu Thr Lys Arg Ser Asn Thr Gln Arg Gln Gln Ile Ala

                          65                   70                   75
      Lys Ser Phe Lys Ala Gln Phe Gly Lys Asp Leu Thr Glu Thr Leu

                          80                   85                   90
      Lys Ser Glu Leu Ser Gly Lys Phe Glu Arg Leu Ile Val Ala Leu

                          95                   100                  105
      Met Tyr Pro Pro Tyr Arg Tyr Glu Ala Lys Glu Leu His Asp Ala

                          110                  115                  120
      Met Lys Gly Leu Gly Thr Lys Glu Gly Val Ile Ile Glu Ile Leu

                          125                  130                  135
      Ala Ser Arg Thr Lys Asn Gln Leu Arg Glu Ile Met Lys Ala Tyr
```

```
                      140                     145                     150
     Glu Glu Asp Tyr Gly Ser Ser Leu Glu Glu Asp Ile Gln Ala Asp


                      155                     160                     165
     Thr Ser Gly Tyr Leu Glu Arg Ile Leu Val Cys Leu Leu Gln Gly


                      170                     175                     180
     Ser Arg Asp Asp Val Ser Ser Phe Val Asp Pro Ala Leu Ala Leu


                      185                     190                     195
     Gln Asp Ala Gln Asp Leu Tyr Ala Ala Gly Glu Lys Ile Arg Gly


                      200                     205                     210
     Thr Asp Glu Met Lys Phe Ile Thr Ile Leu Cys Thr Arg Ser Ala


                      215                     220                     225
     Thr His Leu Leu Arg Val Phe Glu Glu Tyr Glu Lys Ile Ala Asn


                      230                     235                     240
     Lys Ser Ile Glu Asp Ser Ile Lys Ser Glu Thr His Gly Ser Leu


                      245                     250                     255
     Glu Glu Ala Met Leu Thr Val Val Lys Cys Thr Gln Asn Leu His


                      260                     265                     270
     Ser Tyr Phe Ala Glu Arg Leu Tyr Tyr Ala Met Lys Gly Ala Gly


                      275                     280                     285
     Thr Arg Asp Gly Thr Leu Ile Arg Asn Ile Val Ser Arg Ser Glu


                      290                     295                     300
     Ile Asp Leu Asn Leu Ile Lys Cys His Phe Lys Lys Met Tyr Gly


                      305                     310                     315
     Lys Thr Leu Ser Ser Met Ile Met Glu Asp Thr Ser Gly Asp Tyr


                      320                     325
     Lys Asn Ala Leu Leu Ser Leu Val Gly Ser Asp Pro   *
```

entspricht, wobei gegebenenfalls an Position 1 das Methionin abgespalten ist und das Alanin an Position 2 gegebenenfalls blockiert ist und/oder wobei gegebenenfalls intramolekulare Disulfidbrücken zwischen den Cysteinen an den Positionen 161 und/oder 206 und/oder 250 und/oder 293 und/oder Aggregationen durch intermolekulare Disulfidbrücken zwischen den genannten Positionen vorliegen, oder seinen biologisch aktiven Varianten oder Derivaten entspricht.

Besonders bevorzugt sind Mittel, die $Ca^{2+}$ und $Zn^{2+}$ enthalten, wobei sie zwischen 0,01 und 100 mM, vorzugsweise zwischen 0,03 und 10 mM $Ca^{2+}$ und/oder zwischen 0,1 und 100 µM, vorzugsweise zwischen 18 und 30 µM $Zn^{2+}$ enthalten, besonders bevorzugt ist die $Zn^{2+}$ Konzentration im Bereich der normalen Plasma Zink Konzentration.

Die vorliegende Erfindung betrifft weiterhin Verfahren zur Herstellung erfindungsgemäßer Mittel, dadurch gekennzeichnet, daß ein vaskulares Antikoagulanz aus der Gruppe der Annexine und seinen natürlich vorkommenden oder synthetisch oder gentechnisch hergestellten Derivaten oder Analoga mit $Ca^{2+}$ und $Zn^{2+}$ vorzugsweise in den obengenannten Konzentrationen und gegebenenfalls mit Hilfs- und/oder Trägerstoffen und/oder Stabilisatoren versetzt und gegebenenfalls gefriergetrocknet wird.

Außerdem ist auch die Verwendung der erfindungsgemäßen Mittel zur Behandlung und/oder Prophylaxe von Thrombosen oder Embolien oder zur medizinischen Anwendung außerhalb des Organismus Gegenstand der vorliegenden Erfindung.

## Materialien und Methoden

VAC wurde entweder analog EPA 0 181 465 oder EPA 0 293 567 hergestellt. Die nachfolgenden Experimente wurden mit VACα durchgeführt, doch sind die Ergebnisse auch auf die anderen Annexine, insbesondere auch auf VACβ übertragbar.

## Lipide

Dioleoyl-phosphatidylcholin (DOPC, Nr. P-1013)
Dioleoyl-phosphatidylethanolamin (DOPE, Nr. P-0510),
Cardiolipin (CL, Nr. C-5646),
Dioleoyl-phosphatidylglycerol (DOPG, Nr. P-9664),
Phosphatidylinositol (PI, Nr. P-0639),
Dioleoyl-phosphatidsäure (DOPA, Nr. P-2767),
Stearylamin (SA, S-6755) und Eigelb Sphingomyelin (S-0756) wurden bezogen von der Firma Sigma Chemical Co.

Die Reinheit von DOPC und DOPE wurde durch Dünnschichtchromatographie überprüft. Dioleoyl-phosphatidylserin (DOPS) wurde durch Konversion von DOPC gemäß (1) hergestellt. $^{14}C$ markiertes DOPS (spez. Akt. 100.000 dpm/µg) wurde von Amersham bezogen.

## Herstellung der Phospholipid-Doppelschichten auf Siliziumplatten.

Phospholipid Doppelschichten wurden mit einem "Langmuir-film balance" (Lauda Typ FW-1) wie bei Corsel et al. (2) beschrieben aufgetragen. Hydrophile Siliziumplatten wurden 24 Stunden in 30 % Chromschwefelsäure und Wasser behandelt und in 50 % Ethanol/Wasser aufbewahrt. Vor ihrer Verwendung wurden sie gründlich mit einem Detergenz und Wasser gereinigt. Der "film balance" wurde mit demineralisiertem Wasser und 50 µM $CaCl_2$ gefüllt. Auf diese Unterphase wurden 20 µl einer Lösung, die ca. 2g/l Phospholipid in Chloroform enthält, aufgebracht. Die DOPS Fraktionen in den Doppelschichten wurden mit $^{14}C$-markiertem DOPS in Mischung mit DOPC überprüft. Die aufgebauten Doppelschichten wurden von den Siliziumplatten mit dem Szintillations-Detergenz (Du Pont Formel-989) entfernt und die Totalradioaktivität wurde in einem Szintillationszähler gemessen.

## Messung der Bindung durch Ellipsometrie

Die Adsorption von VAC an die Phospholipid-Doppelschichten wurde mit Hilfe eines automatischen Ellipsometers wie beschrieben gemessen (2,3).

Die Bindungsversuche wurden in einer hydrophilen Küvette, die 5 ml eines gerührten Puffers enthielt (0,05 M Tris/HCl; 0,1 M NaCl; pH=7,5; T=20°C) durchgeführt. Die divalenten Kationen wurden als Chloride schrittweise zugegeben.

Bei VAC-Konzentrationen <0,1 µg/ml wurde der Puffer, der die spezifische VAC Konzentration enthielt, kontinuierlich zugegeben, um für VAC eine ausreichende Pufferkapazität zu schaffen.

Aus den kombinierten Polarisier- und Analysier-Daten wurde der refraktive Index und die Dicke d des adsorbierten Films bestimmt (4). Die Menge $\Gamma$ der adsorbierten Proteinschicht wurde aus dem refraktiven Index und der Dicke mit Hilfe einer modifizierten Lorentz-Lorenz Gleichung [1] bestimmt (3,5):

$$[1] \qquad \Gamma = 3d(n^2 - nb^2)/[(n^2 + 2)(r(nb^2 + 2) - v(nb^2 - 1))];$$

nb ist der refraktive Index des Puffers. Die Werte r=0,254 und v=0,71 wurden für die spezifische molare Refraktivität und des partiellen spezifischen Volumens eingesetzt (3).

Ergebnisse

Der Effekt von divalenten Kationen auf die Bindung des VAC an Phospholipide.

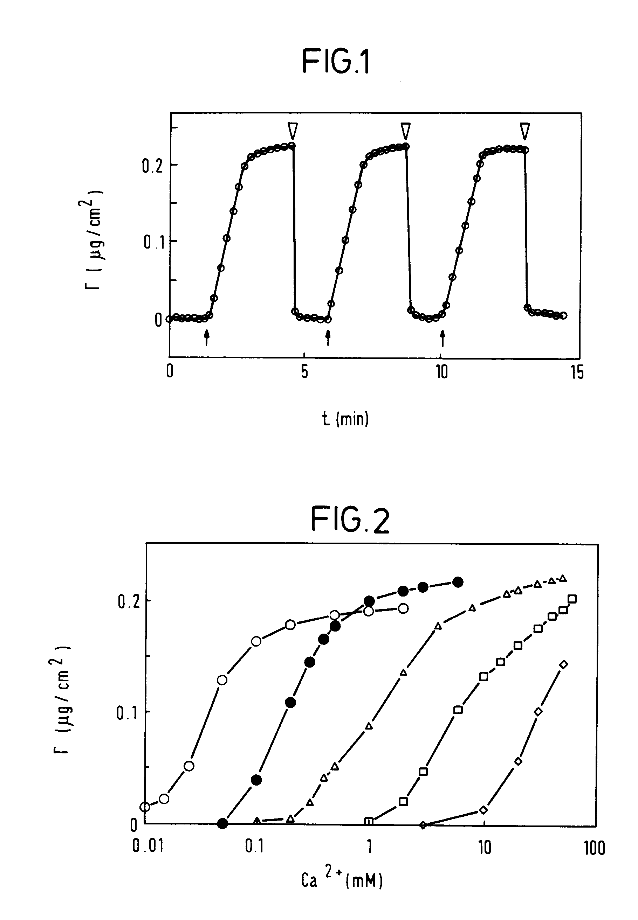

VAC bindet an Phospholipid-Membranen, die aus 20% DOPS/80% DOPC bestehen, in Abhängigkeit von der Calziumkonzentration. Anschließende Zugabe von EDTA führte zur sofortigen und vollständigen Desorption (Fig. 1). Durch die Veränderung der freien $Ca^{2+}$-Konzentration konnte die Adsorption mehrere Male wieder ausgelöst werden, ohne daß die adsorbierte Menge oder die Adsorptionsrate sich merklich veränderte. Irreversible Änderungen des VAC-Moleküls oder der Phospholipid-Doppelschichten durch die Adsorption oder Desorption sind daher nicht wahrscheinlich. Die Bindung war ebenfalls vollständig reversibel, wenn die Küvette mit $Ca^{2+}$-freiem Puffer gespült wurde.

Die $Ca^{2+}$-Abhängigkeit der VAC-Bindung an Phospholipide ist in Fig. 2 dargestellt. Die $Ca^{2+}$-Dosis-Wirkungs-Kurve zeigt ganz eindeutig eine $Ca^{2+}$-Konzentration, bei der die Hälfte der maximalen VAC-Adsorption erreicht wird: $[Ca^{2+}]_{1/2}$. Der $[Ca^{2+}]_{1/2}$-Wert hängt von der Zusammensetzung der Phospholipidoberfläche ab. Bei Phospholipidoberflächen, die 100%, 20%, 5% und 1% DOPS enthalten, wurden $[Ca^{2+})_{1/2}$-Werte von 36 $\mu$M, 220 $\mu$M, 1,5 mM bzw. 8,6 mM gemessen (Tab. 1). Diese Ergebnisse stimmen recht gut überein mit dem $[Ca^{2+}]_{1/2}$-Wert von 53 $\mu$M, der für die Endonexin II (=VAC)- Bindung an äquimolaren Mischungen von PS/PC Vesikeln gemessen wurde (6). Die maximale Menge des adsorbierten Proteins ($\Gamma$max) war unabhängig von der DOPS Fraktion der Membran und betrug ca. 0,217 $\mu$g/cm². 

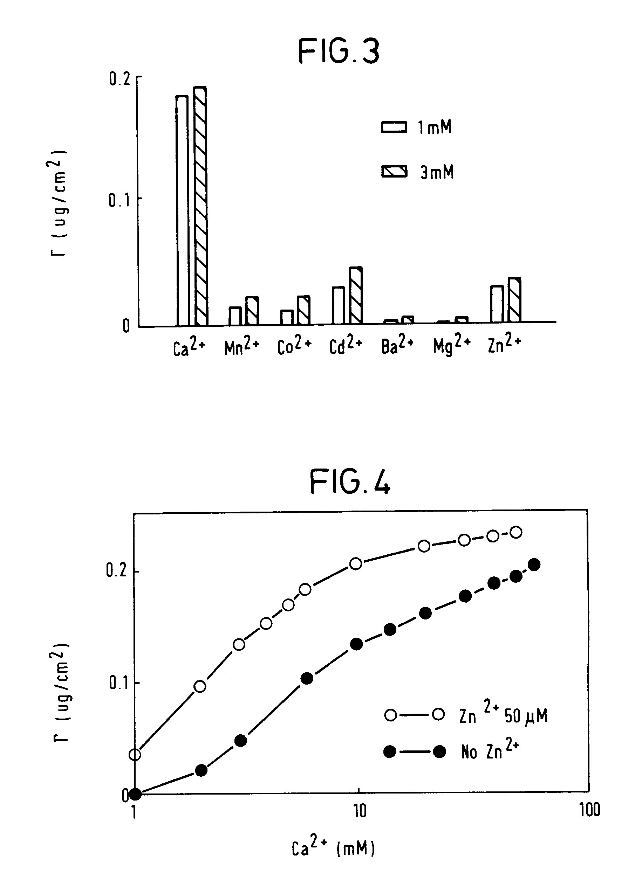

Bei Versuchen mit anderen Kationen als $Ca^{2+}$ wurde festgestellt, daß die Bindung von VAC an die Phospholipide in starkem Maße $Ca^{2+}$-spezifisch ist (Fig. 3). $Cd^{2+}$, $Zn^{2+}$, $Mn^{2+}$ und $Co^{2+}$ zeigten geringe Förderung der Bindung; $Ba^{2+}$ und $Mg^{2+}$ hatten keinen Einfluß. Diese Eigenschaft der Kationen kann in gewisser Weise mit ihren Ionenradien korreliert werden.

Zink Synergismus

Hohe Konzentrationen an Zink-Ionen (1 mM) fördern nur in geringem Maß VAC-Adsorption (Fig. 3); 50 $\mu$M haben überhaupt keinen Einfluß auf die Adsorption. Überraschenderweise beeinflußt diese Konzentration die Bindung in Anwesenheit von $Ca^{2+}$; ein Synergismus ist zu beobachten. Der $[Ca^{2+}]_{1/2}$-Wert fiel von 8,6 auf 2,7 mM für Doppelschichten mit nur 1% DOPS ($[Zn^{2+}]$=50 $\mu$M.) (Fig. 4). 50 $\mu$M $[Zn^{2+}]$ liegt im normalen Bereich der Plasma Zink-Konzentrationen.

Legende zu den Figuren

Fig. 1: Alternierende Adsorption und Desorption von VAC an eine Phospholipidoberfläche, induziert durch Erhöhung bzw. Erniedrigung der $Ca^{2+}$-Konzentration. Die Adsorption von VAC (1 $\mu$g/ml) an eine 20 % DOPS/80 % DOPC Phospholipid-Doppelschicht. Zugabe, von $Ca^{2+}$ (3,4,6 mM) ist durch $\uparrow$ bzw. $\nabla$ gekennzeichnet.

Fig. 2: Einfluß der Phospholipid-Zusammensetzung und der $Ca^{2+}$ Konzentration auf die Adsorption von VAC auf eine Phospholipidoberfläche.
o 100 % DOPS; o 20 % DOPS; $\triangle$ 5 % DOPS; $\square$ 1 % DOPS; 100 % DOPC; sämtliche Mischungen wurden ergänzt mit DOPC. [VAC] = 1 $\mu$g/ml.

Fig. 3: Effekt von bivalenten Ionen auf die Adsorption von VAC. VAC Adsorption an Doppelschichten aus 20 % DOPS und 80 % DOPC in Gegenwart der angegebenen Ionen (1 oder 3 mM). [VAC] = 1 $\mu$g/ml.

Fig. 4: Synergistischer Effekt von $Zn^{2+}$ auf die $Ca^{2+}$-abhängige Adsorption von VAC an die Phospholipidoberfläche.
Der Effekt von $Ca^{2+}$ auf die VAC Adsorption an 1 % DOPS und 99 % DOPC in Gegenwart von 50 $\mu$M $Zn^{2+}$ wurde gemessen. [VAC] = 1 $\mu$g/ml.

Tabelle 1:

Die Hälfte der maximalen VAC-Bindung an verschiedene Phospholipidoberflächen.

| Lipid (mol%/mol%) | $\Gamma$max $\pm$ S.D. ($\mu g/cm^2$) | $[Ca^{2+}]_{1/2}\pm$S.D. mM |
|---|---|---|
| DOPS (100) | $0,195 \pm 0,025$ | $0,036 \pm 0,013$ |
| DOPS / DOPC (20/80) | $0,222 \pm 0,014$ | $0,22 \pm 0,06$ |
| DOPS / DOPC (5/95) | $0,229 \pm 0,004$ | $1,5 \pm 0,5$ |
| DOPS / DOPC (1/99) | $0,234 \pm 0,007$ | $8,6 \pm 2,5$ |
| Cardiolipin / DOPC (20/80) | $0,209 \pm 0,011$ | $0,039 \pm 0,022$ |
| DOPG / DOPC (20/80) | $0,212 \pm 0,003$ | $0,155 \pm 0,027$ |
| PI / DOPC (20/80) | $0,221 \pm 0,005$ | $0,47 \pm 0,05$ |
| DOPA / DOPC (20/80) | $0,207 \pm 0,006$ | $0,75 \pm 0,26$ |
| DOPE / DOPC (20/80) | $0,213 \pm 0,003$ | $0,86 \pm 0,21$ |
| Sphingomyelin / DOPC (20/80) | $0,225 \pm 0,014$ | $7 \pm 3$ |
| DOPC (100) | n.d. | >30 mM |

Die maximale VAC-Adsorption ($\Gamma$max) an die aufgeführten Phospholipidoberflächen zusammen mit der Calzium-Konzentration, die zur Hälfte der maximalen VAC-Bindung $[Ca^{2+}]_{1/2}$ führt, sind als Mittelwerte aus wenigstens drei verschiedenen Experimenten mit den entsprechenden Standard-Abweichungen angegeben.

n.d. = not determined = nicht bestimmt.

Literaturverzeichnis

1. Confurius, P & Zwaal, R. F. A. (1977) Biochim. Biophys. Acta 488, -42.
2. Corsel, J. W., Willems, G. M., Kop, J. M. M., Cuypers, P. A. & Hermens, W. Th. (1986) J. Colloid Interface Sci. 111, 544-554.
3. Cuypers, P. A., Corsel, J. W., Janssen, M. P., Kop, J. M. M., Hermens, W. TH. & Hemker, H. C. (1983) J. Biol. Chem. 258, 2426-2431.
4. McCrackin, F. L., Passaglia, E., Stromberg, R. R. & Steinberg, H. L. (1963) J.Res.Nat.Bur.Stand.Sect.A 67, 3-377.
5. Kop, J. M. M., Cuypers, P. A., Lindhout, Th., Hemker, H. C. & Hermens, W. Th. (1984) J. Biol. Chem. 259, 13993-13998.
6. Schlaepfer, D. D., Mehlman, T., Burgess, W. H. & Haigler. H. T. (1987) Proc. Natl. Acad. Sci. USA 84, 6078-6082.

**Patentansprüche**

1. Mittel, dadurch gekennzeichnet, daß es ein vaskulares Antikoagulanz aus der Gruppe der Annexine und seinen natürlich vorkommenden oder synthetisch oder gentechnisch hergestellten Derivaten oder Analoga und $Ca^{2+}$ und $Zn^{2+}$ als bivalente Kationen und gegebenenfalls Hilfs- und/oder Trägerstoffe und/oder Stabilisatoren enthält.

2. Mittel nach Anspruch 1, dadurch gekennzeichnet, daß das vaskulare Antikoagulanz der Formel

| 1 | | | | 5 | | | | | 10 | | | | | 15 |
|---|---|---|---|---|---|---|---|---|---|---|---|---|---|---|
| Met | Ala | Gln | Val | Leu | Arg | Gly | Thr | Val | Thr | Asp | Phe | Pro | Gly | Phe |

| | | | | 20 | | | | | 25 | | | | | 30 |
|---|---|---|---|---|---|---|---|---|---|---|---|---|---|---|
| Asp | Glu | Arg | Ala | Asp | Ala | XX | Thr | Leu | Arg | Lys | Ala | Met | Lys | Gly |

| | | | | 35 | | | | | 40 | | | | | 45 |
|---|---|---|---|---|---|---|---|---|---|---|---|---|---|---|
| Leu | Gly | Thr | Asp | Glu | Glu | Ser | Ile | Leu | Thr | Leu | Leu | Thr | Ser | Arg |

| | | | | 50 | | | | | 55 | | | | | 60 |
|---|---|---|---|---|---|---|---|---|---|---|---|---|---|---|
| Ser | Asn | Ala | Gln | Arg | Gln | Glu | Ile | Ser | Ala | Ala | Phe | Lys | Thr | Leu |

| | | | | 65 | | | | | 70 | | | | | 75 |
|---|---|---|---|---|---|---|---|---|---|---|---|---|---|---|
| Phe | Gly | Arg | Asp | Leu | Leu | Asp | Asp | Leu | Lys | Ser | Glu | Leu | Thr | Gly |

| | | | | 80 | | | | | 85 | | | | | 90 |
|---|---|---|---|---|---|---|---|---|---|---|---|---|---|---|
| Lys | Phe | Glu | Lys | Leu | Ile | Val | Ala | Leu | Met | Lys | Pro | Ser | Arg | Leu |

```
                   95                    100                   105
Tyr Asp Ala Tyr Glu Leu Lys His Ala Leu Lys Gly Ala Gly Thr

                   110                   115                   120
Asn Glu Lys Val Leu Thr Glu Ile Ile Ala Ser Arg Thr Pro Glu

                   125                   130                   135
Glu Leu Arg Ala Ile Lys Gln Val Tyr Glu Glu Glu Tyr Gly Ser

                   140                   145                   150
Ser Leu Glu Asp Asp Val Val Gly Asp Thr Ser Gly Tyr Tyr Gln

                   155                   160                   165
Arg Met Leu Val Val Leu Leu Gln Ala Asn Arg Asp Pro Asp Ala

                   170                   175                   180
Gly Ile Asp Glu Ala Gln Val Glu Gln Asp Ala Gln Ala Leu Phe

                   185                   190                   195
Gln Ala Gly Glu Leu Lys Trp Gly Thr Asp Glu Glu Lys Phe Ile

                   200                   205                   210
Thr Ile Phe Gly Thr Arg Ser Val Ser His Leu Arg Lys Val Phe

                   215                   220                   225
Asp Lys Tyr Met Thr Ile Ser Gly Phe Gln Ile Glu Glu Thr Ile

                   230                   235                   240
Asp Arg Glu Thr Ser Gly Asn Leu Glu Gln Leu Leu Leu Ala Val

                   245                   250                   255
Val Lys Ser Ile Arg Ser Ile Pro Ala Tyr Leu Ala Glu Thr Leu
```

```
            260                    265                    270
Tyr Tyr Ala Met Lys Gly Ala Gly Thr Asp Asp His Thr Leu Ile


            275                    280                    285
Arg Val Met Val Ser Arg Ser Glu Ile Asp Leu Phe Asn Ile Arg


            290                    295                    300
Lys Glu Phe Arg Lys Asn Phe Ala Thr Ser Leu Tyr Ser Met Ile


            305                    310                    315
Lys Gly Asp Thr Ser Gly Asp Tyr Lys Lys Ala Leu Leu Leu Leu


            320
Cys Gly Glu Asp Asp    *
```

entspricht, wobei XX für Glu oder Asp steht und gegebenenfalls an Position 1 das Methionin abgespalten ist und das Alanin an Position 2 gegebenenfalls blockiert ist und/oder wobei gegebenenfalls Aggregationen durch beispielsweise intermolekulare Disulfidbrücken zwischen den Cysteinen an Position 316 vorliegen oder seinen biologisch aktiven Varianten oder Derivaten entspricht oder daß es der Formel

```
  1                     5                     10                     15
Met Ala Trp Trp Lys Ala Trp Ile Glu Gln Glu Gly Val Thr Val


            20                     25                     30
Lys Ser Ser Ser His Phe Asn Pro Asp Pro Asp Ala Glu Thr Leu


            35                     40                     45
Tyr Lys Ala Met Lys Gly Ile Gly Thr Asn Glu Gln Ala Ile Ile


            50                     55                     60
Asp Val Leu Thr Lys Arg Ser Asn Thr Gln Arg Gln Gln Ile Ala
```

<table>
<tr><td></td><td></td><td>65</td><td></td><td></td><td></td><td></td><td>70</td><td></td><td></td><td></td><td></td><td>75</td></tr>
<tr><td colspan="13">Lys Ser Phe Lys Ala Gln Phe Gly Lys Asp Leu Thr Glu Thr Leu</td></tr>
</table>

<table>
<tr><td></td><td></td><td>80</td><td></td><td></td><td></td><td></td><td>85</td><td></td><td></td><td></td><td></td><td>90</td></tr>
<tr><td colspan="13">Lys Ser Glu Leu Ser Gly Lys Phe Glu Arg Leu Ile Val Ala Leu</td></tr>
</table>

<table>
<tr><td></td><td></td><td>95</td><td></td><td></td><td></td><td></td><td>100</td><td></td><td></td><td></td><td></td><td>105</td></tr>
<tr><td colspan="13">Met Tyr Pro Pro Tyr Arg Tyr Glu Ala Lys Glu Leu His Asp Ala</td></tr>
</table>

<table>
<tr><td></td><td></td><td>110</td><td></td><td></td><td></td><td></td><td>115</td><td></td><td></td><td></td><td></td><td>120</td></tr>
<tr><td colspan="13">Met Lys Gly Leu Gly Thr Lys Glu Gly Val Ile Ile Glu Ile Leu</td></tr>
</table>

<table>
<tr><td></td><td></td><td>125</td><td></td><td></td><td></td><td></td><td>130</td><td></td><td></td><td></td><td></td><td>135</td></tr>
<tr><td colspan="13">Ala Ser Arg Thr Lys Asn Gln Leu Arg Glu Ile Met Lys Ala Tyr</td></tr>
</table>

<table>
<tr><td></td><td></td><td>140</td><td></td><td></td><td></td><td></td><td>145</td><td></td><td></td><td></td><td></td><td>150</td></tr>
<tr><td colspan="13">Glu Glu Asp Tyr Gly Ser Ser Leu Glu Glu Asp Ile Gln Ala Asp</td></tr>
</table>

<table>
<tr><td></td><td></td><td>155</td><td></td><td></td><td></td><td></td><td>160</td><td></td><td></td><td></td><td></td><td>165</td></tr>
<tr><td colspan="13">Thr Ser Gly Tyr Leu Glu Arg Ile Leu Val Cys Leu Leu Gln Gly</td></tr>
</table>

<table>
<tr><td></td><td></td><td>170</td><td></td><td></td><td></td><td></td><td>175</td><td></td><td></td><td></td><td></td><td>180</td></tr>
<tr><td colspan="13">Ser Arg Asp Asp Val Ser Ser Phe Val Asp Pro Ala Leu Ala Leu</td></tr>
</table>

<table>
<tr><td></td><td></td><td>185</td><td></td><td></td><td></td><td></td><td>190</td><td></td><td></td><td></td><td></td><td>195</td></tr>
<tr><td colspan="13">Gln Asp Ala Gln Asp Leu Tyr Ala Ala Gly Glu Lys Ile Arg Gly</td></tr>
</table>

<table>
<tr><td></td><td></td><td>200</td><td></td><td></td><td></td><td></td><td>205</td><td></td><td></td><td></td><td></td><td>210</td></tr>
<tr><td colspan="13">Thr Asp Glu Met Lys Phe Ile Thr Ile Leu Cys Thr Arg Ser Ala</td></tr>
</table>

<table>
<tr><td></td><td></td><td>215</td><td></td><td></td><td></td><td></td><td>220</td><td></td><td></td><td></td><td></td><td>225</td></tr>
<tr><td colspan="13">Thr His Leu Leu Arg Val Phe Glu Glu Tyr Glu Lys Ile Ala Asn</td></tr>
</table>

<table>
<tr><td></td><td></td><td>230</td><td></td><td></td><td></td><td></td><td>235</td><td></td><td></td><td></td><td></td><td>240</td></tr>
<tr><td colspan="13">Lys Ser Ile Glu Asp Ser Ile Lys Ser Glu Thr His Gly Ser Leu</td></tr>
</table>

<pre>
                  245                 250                 255
  Glu Glu Ala Met Leu Thr Val Val Lys Cys Thr Gln Asn Leu His

                  260                 265                 270
  Ser Tyr Phe Ala Glu Arg Leu Tyr Tyr Ala Met Lys Gly Ala Gly

                  275                 280                 285
  Thr Arg Asp Gly Thr Leu Ile Arg Asn Ile Val Ser Arg Ser Glu

                  290                 295                 300
  Ile Asp Leu Asn Leu Ile Lys Cys His Phe Lys Lys Met Tyr Gly

                  305                 310                 315
  Lys Thr Leu Ser Ser Met Ile Met Glu Asp Thr Ser Gly Asp Tyr

                  320                 325
  Lys Asn Ala Leu Leu Ser Leu Val Gly Ser Asp Pro   *
</pre>

entspricht, wobei gegebenenfalls an Position 1 das Methionin abgespalten ist und das Alanin an Position 2 gegebenenfalls blockiert ist und/oder wobei gegebenenfalls intramolekulare Disulfidbrücken zwischen den Cysteinen an den Positionen 161 und/oder 206 und/oder 250 und/oder 293 und/oder Aggregationen durch intermolekulare Disulfidbrücken zwischen den genannten Positionen vorliegen, oder seinen biologisch aktiven Varianten oder Derivaten entspricht.

3. Mittel nach einem der Ansprüche 1 oder 2, dadurch gekennzeichnet, daß es zwischen 0,01 und 100 mM, vorzugsweise zwischen 0,03 und 10 mM $Ca^{2+}$ enthält.

4. Mittel nach einem der Ansprüche 1 bis 3, dadurch gekennzeichnet, daß es zwischen 0,1 und 100 $\mu$M, vorzugsweise zwischen 18 und 30 $\mu$M $Zn^{2+}$ enthält.

5. Mittel nach Anspruch 4, dadurch gekennzeichnet, daß die $Zn^{2+}$ Konzentration im Bereich der normalen Plasma Zink Konzentration liegt.

6. Mittel nach Anspruch 5, dadurch gekennzeichnet, daß es zwischen 0,01 und 100 mM, vorzugsweise zwischen 0,03 und 10 mM $Ca^{2+}$ und zwischen 0,1 und 100 $\mu$M, vorzugsweise zwischen 1,8 und 30 $\mu$M, besonders bevorzugt im Bereich der normalen Plasma Zink Konzentration, $Zn^{2+}$ enthält.

7. Verfahren zur Herstellung eines Mittels nach Anspruch 1, dadurch gekennzeichnet, daß ein vaskulares Antikoagulanz aus der Gruppe der Annexine und seinen natürlich vorkommenden oder synthetisch oder gentechnisch hergestellten Derivaten oder Analoga und $Ca^{2+}$ und $Zn^{2+}$ als bivalente Kationen, vorzugsweise in Konzentration wie in den Ansprüchen 3 bis 5 angegeben und gegebenenfalls mit Hilfs- und/oder Trägerstoffen und/oder Stabilisatoren versetzt und gegebenenfalls gefriergetrocknet wird.

8. Mittel nach einem der Ansprüche 1 bis 6 zur Verwendung als Arzneimittel.

9. Mittel nach einem der Ansprüche 1 bis 6 zur Behandlung und/oder Prophylaxe von Thrombosen der Embolien.

10. Verwendung eines Mittels nach einem der Ansprüche 1 bis 6 zur medizinischen Anwendung außerhalb des Organismus.

**Claims**

1. Agent, characterised in that it contains a vascular anticoagulant selected from the group of the annexins and the naturally occurring or synthetically produced or genetically engineered derivatives or analogs thereof and Ca$^{2+}$ and Zn$^{2+}$ as bivalent cations and optionally excipients and/or carriers and/or stabilisers.

2. Agent according to claim 1, characterised in that the vascular anticoagulant corresponds to the formula

```
     1                 5                 10                15
   Met Ala Gln Val Leu Arg Gly Thr Val Thr Asp Phe Pro Gly Phe

                      20                25                30
   Asp Glu Arg Ala Asp Ala XX  Thr Leu Arg Lys Ala Met Lys Gly

                      35                40                45
   Leu Gly Thr Asp Glu Glu Ser Ile Leu Thr Leu Leu Thr Ser Arg

                      50                55                60
   Ser Asn Ala Gln Arg Gln Glu Ile Ser Ala Ala Phe Lys Thr Leu

                      65                70                75
   Phe Gly Arg Asp Leu Leu Asp Asp Leu Lys Ser Glu Leu Thr Gly

                      80                85                90
   Lys Phe Glu Lys Leu Ile Val Ala Leu Met Lys Pro Ser Arg Leu
```

```
                95                    100                   105
Tyr Asp Ala Tyr Glu Leu Lys His Ala Leu Lys Gly Ala Gly Thr

                110                   115                   120
Asn Glu Lys Val Leu Thr Glu Ile Ile Ala Ser Arg Thr Pro Glu

                125                   130                   135
Glu Leu Arg Ala Ile Lys Gln Val Tyr Glu Glu Glu Tyr Gly Ser

                140                   145                   150
Ser Leu Glu Asp Asp Val Val Gly Asp Thr Ser Gly Tyr Tyr Gln

                155                   160                   165
Arg Met Leu Val Val Leu Leu Gln Ala Asn Arg Asp Pro Asp Ala

                170                   175                   180
Gly Ile Asp Glu Ala Gln Val Glu Gln Asp Ala Gln Ala Leu Phe

                185                   190                   195
Gln Ala Gly Glu Leu Lys Trp Gly Thr Asp Glu Glu Lys Phe Ile

                200                   205                   210
Thr Ile Phe Gly Thr Arg Ser Val Ser His Leu Arg Lys Val Phe

                215                   220                   225
Asp Lys Tyr Met Thr Ile Ser Gly Phe Gln Ile Glu Glu Thr Ile

                230                   235                   240
Asp Arg Glu Thr Ser Gly Asn Leu Glu Gln Leu Leu Leu Ala Val

                245                   250                   255
Val Lys Ser Ile Arg Ser Ile Pro Ala Tyr Leu Ala Glu Thr Leu
```

```
                 260                      265                       270
Tyr Tyr Ala Met Lys Gly Ala Gly Thr Asp Asp His Thr Leu Ile

                 275                      280                       285
Arg Val Met Val Ser Arg Ser Glu Ile Asp Leu Phe Asn Ile Arg

                 290                      295                       300
Lys Glu Phe Arg Lys Asn Phe Ala Thr Ser Leu Tyr Ser Met Ile

                 305                      310                       315
Lys Gly Asp Thr Ser Gly Asp Tyr Lys Lys Ala Leu Leu Leu Leu

                 320
Cys Gly Glu Asp Asp    *
```

wherein XX represents Glu or Asp and optionally at position 1 the methionine is cleaved and the alanine at position 2 is optionally blocked and/or there are optionally aggregations caused by intermolecular disulphide bridges, for example, between the cysteines at position 316, or corresponds to the biologically active variants or derivatives thereof, or in that it corresponds to the formula

```
    1                  5                        10                        15
Met Ala Trp Trp Lys Ala Trp Ile Glu Gln Glu Gly Val Thr Val

                 20                       25                        30
Lys Ser Ser Ser His Phe Asn Pro Asp Pro Asp Ala Glu Thr Leu

                 35                       40                        45
Tyr Lys Ala Met Lys Gly Ile Gly Thr Asn Glu Gln Ala Ile Ile

                 50                       55                        60
Asp Val Leu Thr Lys Arg Ser Asn Thr Gln Arg Gln Gln Ile Ala
```

                    65                        70                        75
Lys Ser Phe Lys Ala Gln Phe Gly Lys Asp Leu Thr Glu Thr Leu

                    80                        85                        90
Lys Ser Glu Leu Ser Gly Lys Phe Glu Arg Leu Ile Val Ala Leu

                    95                        100                       105
Met Tyr Pro Pro Tyr Arg Tyr Glu Ala Lys Glu Leu His Asp Ala

                    110                       115                       120
Met Lys Gly Leu Gly Thr Lys Glu Gly Val Ile Ile Glu Ile Leu

                    125                       130                       135
Ala Ser Arg Thr Lys Asn Gln Leu Arg Glu Ile Met Lys Ala Tyr

                    140                       145                       150
Glu Glu Asp Tyr Gly Ser Ser Leu Glu Glu Asp Ile Gln Ala Asp

                    155                       160                       165
Thr Ser Gly Tyr Leu Glu Arg Ile Leu Val Cys Leu Leu Gln Gly

                    170                       175                       180
Ser Arg Asp Asp Val Ser Ser Phe Val Asp Pro Ala Leu Ala Leu

                    185                       190                       195
Gln Asp Ala Gln Asp Leu Tyr Ala Ala Gly Glu Lys Ile Arg Gly

                    200                       205                       210
Thr Asp Glu Met Lys Phe Ile Thr Ile Leu Cys Thr Arg Ser Ala

                    215                       220                       225
Thr His Leu Leu Arg Val Phe Glu Glu Tyr Glu Lys Ile Ala Asn

                    230                       235                       240
Lys Ser Ile Glu Asp Ser Ile Lys Ser Glu Thr His Gly Ser Leu

```
                    245                         250                         255
    Glu Glu Ala Met Leu Thr Val Val Lys Cys Thr Gln Asn Leu His

                    260                         265                         270
    Ser Tyr Phe Ala Glu Arg Leu Tyr Tyr Ala Met Lys Gly Ala Gly

                    275                         280                         285
    Thr Arg Asp Gly Thr Leu Ile Arg Asn Ile Val Ser Arg Ser Glu

                    290                         295                         300
    Ile Asp Leu Asn Leu Ile Lys Cys His Phe Lys Lys Met Tyr Gly

                    305                         310                         315
    Lys Thr Leu Ser Ser Met Ile Met Glu Asp Thr Ser Gly Asp Tyr

                    320                         325
    Lys Asn Ala Leu Leu Ser Leu Val Gly Ser Asp Pro    *
```

wherein optionally at position 1 the methionine is cleaved and the alanine at position 2 is optionally blocked and/or there are optionally intramolecular disulphide bridges between the cysteines at positions 161 and/or 206 and/or 250 and/or 293 and/or aggregations caused by intermolecular disulphide bridges between the said positions, or corresponds to the biologically active variants or derivatives thereof.

3. Agent according to one of claims 1 and 2, characterised in that it contains between 0.01 and 100 mM, preferably between 0.03 and 10 mM $Ca^{2+}$.

4. Agent according to one of claims 1 to 3, characterised in that it contains between 0.1 and 100 $\mu$M, preferably between 18 and 30 $\mu$M $Zn^{2+}$.

5. Agent according to claim 4, characterised in that the $Zn^{2+}$ concentration is within the range of the normal plasma zinc concentration.

6. Agent according to claim 5, characterised in that it contains between 0.01 and 100 mM, preferably between 0.03 and 10 mM $Ca^{2+}$ and between 0.1 and 100 $\mu$M, preferably between 1.8 and 30 $\mu$M of $Zn^{2+}$, particularly preferably in the range of the normal plasma zinc concentration.

7. Process for preparing an agent according to claim 1, characterised in that a vascular anticoagulant from the group of annexins and the naturally occurring or synthetically produced or genetically engineered derivatives or analogs thereof and $Ca^{2+}$ and $Zn^{2+}$ as bivalent cations, preferably in concentrations as specified in claims 3 to 5, are mixed, optionally with excipients and/or carriers and/or stabilisers, and optionally freeze-dried.

8. Agent according to one of claims 1 to 6 for use as a pharmaceutical composition.

9. Agent according to one of claims 1 to 6 for the treatment and/or prophylaxis of thrombosis or embolism.

10. Use of an agent according to one of claims 1 to 6 for medical use outside the body.

**Revendications**

1. Agent caractérisé en ce qu'il contient un anticoagulant vasculaire du groupe des annexines et de leurs dérivés ou analogues naturels, synthétiques ou préparés par génie génétique, Ca$^{2+}$ et Zn$^{2+}$ en tant que cations divalents, et éventuellement des adjuvants et/ou véhicules et/ou stabilisants.

2. Agent selon la revendication 1, caractérisé en ce que l'anticoagulant vasculaire répond à la formule

```
    1                 5                         10                        15
   Met Ala Gln Val Leu Arg Gly Thr Val Thr Asp Phe Pro Gly Phe


                     20                        25                        30
   Asp Glu Arg Ala Asp Ala  XX  Thr Leu Arg Lys Ala Met Lys Gly


                     35                        40                        45
   Leu Gly Thr Asp Glu Glu Ser Ile Leu Thr Leu Leu Thr Ser Arg


                     50                        55                        60
   Ser Asn Ala Gln Arg Gln Glu Ile Ser Ala Ala Phe Lys Thr Leu


                     65                        70                        75
   Phe Gly Arg Asp Leu Leu Asp Asp Leu Lys Ser Glu Leu Thr Gly


                     80                        85                        90
   Lys Phe Glu Lys Leu Ile Val Ala Leu Met Lys Pro Ser Arg Leu


                     95                        100                       105
   Tyr Asp Ala Tyr Glu Leu Lys His Ala Leu Lys Gly Ala Gly Thr


                     110                       115                       120
   Asn Glu Lys Val Leu Thr Glu Ile Ile Ala Ser Arg Thr Pro Glu
```

```
                    125                 130                 135
Glu Leu Arg Ala Ile Lys Gln Val Tyr Glu Glu Glu Tyr Gly Ser


                    140                 145                 150
Ser Leu Glu Asp Asp Val Val Gly Asp Thr Ser Gly Tyr Tyr Gln


                    155                 160                 165
Arg Met Leu Val Val Leu Leu Gln Ala Asn Arg Asp Pro Asp Ala


                    170                 175                 180
Gly Ile Asp Glu Ala Gln Val Glu Gln Asp Ala Gln Ala Leu Phe


                    185                 190                 195
Gln Ala Gly Glu Leu Lys Trp Gly Thr Asp Glu Glu Lys Phe Ile


                    200                 205                 210
Thr Ile Phe Gly Thr Arg Ser Val Ser His Leu Arg Lys val Phe


                    215                 220                 225
Asp Lys Tyr Met Thr Ile Ser Gly Phe Gln Ile Glu Glu Thr Ile


                    230                 235                 240
Asp Arg Glu Thr Ser Gly Asn Leu Glu Gln Leu Leu Leu Ala Val


                    245                 250                 255
Val Lys Ser Ile Arg Ser Ile Pro Ala Tyr Leu Ala Glu Thr Leu


                    260                 265                 270
Tyr Tyr Ala Met Lys Gly Ala Gly Thr Asp Asp His Thr Leu Ile


                    275                 280                 285
Arg val Met Val Ser Arg Ser Glu Ile Asp Leu Phe Asn Ile Arg
```

```
                    290                        295                        300
    Lys Glu Phe Arg Lys Asn Phe Ala Thr Ser Leu Tyr Ser Met Ile


                    305                        310                        315
    Lys Gly Asp Thr Ser Gly Asp Tyr Lys Lys Ala Leu Leu Leu Leu


                    320
    Cys Gly Glu Asp Asp *
```

dans laquelle XX représente Glu ou Asp et la méthionine en position 1 est éventuellement éliminée et l'alanine en position 2 est éventuellement bloquée et/ou dans laquelle il existe éventuellement des agrégations par exemple par des ponts disulfure intermoléculaires entre les cystéines en position 316 ou ses variantes ou dérivés actifs du point de vue biologique, ou en ce qu'il répond à la formule

```
    1                    5                        10                        15
    Met Ala Trp Trp Lys Ala Trp Ile Glu Gln Glu Gly Val Thr Val


                    20                        25                        30
    Lys Ser Ser Ser His Phe Asn Pro Asp Pro Asp Ala Glu Thr Leu


                    35                        40                        45
    Tyr Lys Ala Met Lys Gly Ile Gly Thr Asn Glu Gln Ala Ile Ile


                    50                        55                        60
    Asp Val Leu Thr Lys Arg Ser Asn Thr Gln Arg Gln Gln Ile Ala


                    65                        70                        75
    Lys Ser Phe Lys Ala Gln Phe Gly Lys Asp Leu Thr Glu Thr Leu


                    80                        85                        90
    Lys Ser Glu Leu Ser Gly Lys Phe Glu Arg Leu Ile Val Ala Leu
```

```
              95                    100                   105
Met Tyr Pro Pro Tyr Arg Tyr Glu Ala Lys Glu Leu His Asp Ala


              110                   115                   120
Met Lys Gly Leu Gly Thr Lys Glu Gly Val Ile Ile Glu Ile Leu


              125                   130                   135
Ala Ser Arg Thr Lys Asn Gln Leu Arg Glu Ile Met Lys Ala Tyr


              140                   145                   150
Glu Glu Asp Tyr Gly Ser Ser Leu Glu Glu Asp Ile Gln Ala Asp


              155                   160                   165
Thr Ser Gly Tyr Leu Glu Arg Ile Leu Val Cys Leu Leu Gln Gly


              170                   175                   180
Ser Arg Asp Asp Val Ser Ser Phe Val Asp Pro Ala Leu Ala Leu


              185                   190                   195
Gln Asp Ala Gln Asp Leu Tyr Ala Ala Gly Glu Lys Ile Arg Gly


              200                   205                   210
Thr Asp Glu Met Lys Phe Ile Thr Ile Leu Cys Thr Arg Ser Ala


              215                   220                   225
Thr His Leu Leu Arg Val Phe Glu Glu Tyr Glu Lys Ile Ala Asn


              230                   235                   240
Lys Ser Ile Glu Asp Ser Ile Lys Ser Glu Thr His Gly Ser Leu


              245                   250                   255
Glu Glu Ala Met Leu Thr Val Val Lys Cys Thr Gln Asn Leu His
```

```
            260                    265                    270
Ser Tyr Phe Ala Glu Arg Leu Tyr Tyr Ala Met Lys Gly Ala Gly

            275                    280                    285
Thr Arg Asp Gly Thr Leu Ile Arg Asn Ile Val Ser Arg Ser Glu

            290                    295                    300
Ile Asp Leu Asn Leu Ile Lys Cys His Phe Lys Lys Met Tyr Gly

            305                    310                    315
Lys Thr Leu Ser Ser Met Ile Met Glu Asp Thr Ser Gly Asp Tyr

            320                    325
Lys Asn Ala Leu Leu Ser Leu Val Gly Ser Asp Pro *
```

dans laquelle la méthionine en position 1 est éventuellement éliminée et l'alanine en position 2 est éventuellement bloquée et/ou dans laquelle il existe éventuellement des ponts disulfure intramoléculaires entre les cystéines aux positions 161 et/ou 206 et/ou 250 et/ou 293 et/ou des agrégations par des ponts disulfure intermoléculaires entre les positions citées, ou ses variantes ou dérivés actifs du point de vue biologique.

3. Agent selon l'une des revendications 1 ou 2, caractérisé en ce qu'il contient entre 0,01 et 100 $\mu$M, de préférence entre 0,03 et 10 $\mu$M de $Ca^{2+}$.

4. Agent selon l'une des revendications 1 à 3, caractérisé en ce qu'il contient entre 0,1 et 100 $\mu$M, de préférence entre 18 et 30 $\mu$M de $Zn^{2+}$.

5. Agent selon la revendication 4, caractérisé en ce que la concentration en $Zn^{2+}$ est située dans le domaine de la concentration normale du zinc dans le plasma.

6. Agent selon la revendication 5, caractérisé en ce qu'il contient entre 0,01 et 100 mM, de préférence entre 0,03 et 10 mM de $Ca^{2+}$, et $Zn^{2+}$ en une concentration comprise entre 0,1 et 100 $\mu$M, de préférence entre 1,8 et 30 $\mu$M, de préférence encore dans le domaine de la concentration normale du zinc dans le plasma.

7. Procédé de préparation d'un agent selon la revendication 1, caractérisé en ce qu'un anticoagulant vasculaire du groupe des annexines et de leurs dérivés ou analogues naturels, synthétiques ou préparés par génie génétique est additionné de $Ca^{2+}$ et de $Zn^{2+}$ en tant que cations divalents, de préférence en une concentration telle qu'indiquée dans les revendications 3 à 5 et éventuellement d'adjuvants et/ou de véhicules et/ou de stabilisants et est éventuellement lyophilisé.

8. Agent selon l'une des revendications 1 à 6 destiné à être utilisé comme médicament.

9. Agent selon l'une des revendications 1 à 6 destiné au traitement et/ou à la prophylaxie des thromboses et des embolies.

10. Utilisation d'un agent selon l'une des revendications 1 à 6 en vue d'un emploi médical à l'extérieur de l'organisme.

# FIG.1

# FIG.2

# FIG.3

# FIG.4